# EUROPÄISCHE PATENTSCHRIFT

(11) **EP 1 803 398 B1**
(45) Veröffentlichungstag und Bekanntmachung des Hinweises auf die Patenterteilung: **14.07.2010**
(21) Anmeldenummer: 06016644.4
(22) Anmeldetag: 09.08.2006
(51) Int. Cl.: A61B 6/00, G01N 23/04, H01J 35/08, A61B 6/03

(54) **Fokus-Detektor-Anordnung zur Erzeugung von Phasenkontrast-Röntgenaufnahmen und Verfahren hierzu**
Source-detector arrangement for X-ray phase contrast imaging and method therefor
Agencement d'une source et d'un détecteur pour l'imagerie à rayons X à contraste de phase et procédé associé

(30) Priorität: 27.12.2005 DE 102005062447; 27.12.2005 DE 102005062448; 01.02.2006 DE 102006004604; 01.02.2006 DE 102006004976
(43) Veröffentlichungstag der Anmeldung: 04.07.2007
(73) Patentinhaber: SIEMENS AKTIENGESELLSCHAFT, 80333 München (DE); Paul Scherrer Institut PSI, 5232 Villigen PSI (CH)
(72) Erfinder: Baumann, Joachim, Dr., 81925 München (DE); Schuster, Manfred, Dr., 80686 München (DE); Freudenberger, Jörg, Dr., 90562 Kalchreuth (DE); Hempel, Eckhard, Dr., 90765 Fürth (DE); Hoheisel, Martin, Dr., 91056 Erlangen (DE); Mertelmeier, Thomas, Dr., 91058 Erlangen (DE); Popescu, Stefan, Prof., 91056 Erlangen (DE); David, Christian, Dr., 79787 Lauchringen (DE); Pfeiffer, Franz, Prof., 85748 Garching (DE); Engelhardt, Martin, 81247 München (DE)
(74) Vertreter: Maier, Daniel Oliver

(56) Entgegenhaltungen:
- EP-A- 1 447 046
- WO-A-2004/058070
- DE-A1- 19 510 047
- US-A- 4 057 745
- US-A- 4 723 263
- US-A- 5 812 629
- WEITKAMP T ET AL: "Hard x-ray phase imaging and tomography with a grating interferometer" PROCEEDINGS OF THE SPIE, SPIE, BELLINGHAM, VA, US, Bd. 5535, Nr. 1, Oktober 2004 (2004-10), Seiten 137-142, XP002397630 ISSN: 0277-786X
- MOMOSE A ET AL: "X-ray Talbot interferometry for medical phase imaging" AIP CONFERENCE PROCEEDINGS, AMERICAN INSTITUTE OF PHYSICS, NEW YORK,NY, US, Nr. 716, 13. Januar 2004 (2004-01-13), Seiten 156-159, XP002351238 ISSN: 0094-243X
- PFEIFFER ET AL.: "Phase retrieval and differential phase-contrast imaging with low-brilliance X-ray sources"[Online] 26. März 2006 (2006-03-26), XP002422783 Gefunden im Internet: URL:http://sls.web.psi.ch/view.php/beamlin es/cs/publications/nphys265.pdf> [gefunden am 2007-03-01]

## Beschreibung

Die Erfindung betrifft eine Fokus-Detektor-Anordnung einer Röntgenapparatur zur Erzeugung projektiver oder tomographischer Phasenkontrastaufnahmen von einem Untersuchungsobjekt mit einer auf einer ersten Seite des Untersuchungsobjektes angeordneten Strahlungsquelle, welche ein Bündel von kohärenten Strahlen mit gitterartigem Ursprung erzeugt, einem auf der gegenüberliegenden zweiten Seite des Untersuchungsobjektes im Strahlengang angeordnetes Phasengitter, welches ein Interferenzmuster der Röntgenstrahlung in einem vorbestimmten Energiebereich der Röntgenstrahlung erzeugt, und ein Analyse-Detektor-System, welches zumindest das vom Phasengitter erzeugte Interferenzmuster örtlich bezüglich seiner Phasenverschiebung detektiert. Außerdem betrifft die Erfindung auch ein Verfahren zur Erzeugung projektiver oder tomographischer Röntgen-Phasenkontrastaufnahmen mit der oben genannten Fokus-Detektor-Anordnung.

Solche Fokus-Detektor-Anordnungen zur Erzeugung projektiver oder tomographischer Phasenkontrastaufnahmen von einem Untersuchungsobjekt beziehungsweise solche Verfahren sind allgemein bekannt. Beispielhaft wird auf die EP 1 447 046 A1 und die nicht vorveröffentlichten deutschen Patentanmeldungen mit den Aktenzeichen 10 2006 017 290.6, 10 2006 015 358.8, 10 2006 017 291.4, 10 2006 015 356.1 und 10 2006 015 355.3 verwiesen. Die Druckschrift "Hard X-ray phase imaging and tomography with a grating interferometer" von Weitkamp et al. stellt den nächstkommenden Stand der Technik zu dem unabhängigen Anspruch 1 dar.

Für die Bildgebung durch ionisierende Strahlen, insbesondere durch Röntgenstrahlen können grundsätzlich zwei Effekte betrachtet werden, die beim Durchtritt der Strahlung durch Materie auftreten, nämlich die Absorption und die Phasenverschiebung der durch ein Untersuchungsobjekt durchtretenden Strahlung. Es ist auch bekannt, dass der Effekt der Phasenverschiebungen beim Durchtritt eines Strahles durch ein Untersuchungsobjekt wesentlich stärker auf geringere Unterschiede in der Zusammensetzung der durchdrungenen Materie reagiert, als die Absorptionseffekte.

Für eine solche Phasenkontrastradiographie oder Phasenkontrasttomographie muss die vom Objekt verursachte Phasenverschiebung ausgewertet werden. Hierbei können analog zur Röntgenradiographie beziehungsweise Röntgentomographie sowohl projektive Bilder der Phasenverschiebung oder auch eine Vielzahl von projektiven Bildern tomographischer Darstellungen der Phasenverschiebung, die durch ein Volumenelement bewirkt wird, berechnet werden.

Derartige Phasenverschiebungen zur Erzeugung projektiver oder tomographischer Aufnahmen können durch die Verwendung durch interferometrische Gitter gemessen werden. Bezüglich dieser interferometrischen Messmethoden wird ebenfalls auf die zuvor zitierte Schriften verwiesen. Bei diesen Methoden wird ein Untersuchungsobjekt von einer kohärenten Röntgenstrahlung durchstrahlt, anschließend durch ein Gitter mit einer auf die Wellenlängen der Strahlung angepassten Periode geführt, wodurch ein Interferenzmuster entsteht, welches abhängig von der auftretenden Strahlungsverschiebung ist. Dieses Interferenzmuster wird durch eine anschließende Analyse-Detektor-Anordnung ausgemessen, so dass die Phasenverschiebung bestimmt werden kann.

Das oben beschriebene Verfahren erfordert einen ausreichenden Grad an räumlicher Kohärenz in der verwendeten Strahlung. Dies kann beispielsweise durch einen extrem kleinen Fokus erreicht werden, wobei hier die erreichbare Dosisleistung wegen der zu langen erforderlichen Bestrahlungszeit für medizinische Anwendungen kaum nutzbar ist. Eine andere Möglichkeit ist die Verwendung von Synchrotronstrahlung. Derartige Apparaturen sind in der Praxis viel zu aufwendig. Schließlich wird im genannten Stand der Technik auch vorgeschlagen, einen Fokus mit einem konventionell großen Brennfleck zu verwenden, wie er im Bereich der bekannten Computertomographie bekannt ist, und zwischen Fokus und Untersuchungsobjekt ein sogenanntes Quellengitter anzuordnen. Die Schlitze dieses Quellengitters erzeugen ein Feld von individuell kohärenten Strahlen einer bestimmten Energie, deren Dosisleistung ausreicht, um mit Hilfe eines in Strahlrichtung hinter dem Objekt angeordneten Phasengitter das an sich bekannte Interferenzmuster zu erzeugen.

Auf diese Weise ist es möglich, Strahlungsquellen zu verwenden, die Ausdehnungen besitzen, die normalen Röntgenröhren in CT-Systemen beziehungsweise Durchlicht-Röntgen-Systemen entsprechen, so dass zum Beispiel im Bereich der allgemeinen medizinischen Diagnostik nun mit Hilfe von Röntgen-Geräten auch gut differenzierte Weichteilaufnahmen gemacht werden können.

Ein Problem bei dieser Art von Fokus-Detektor-Kombinationen besteht darin, dass bei der Verwendung solcher Quellengitter trotzdem noch ein relativ hoher Dosisanteil durch Strahlung entsteht, die nicht zur quasi-kohärenten Strahlung zählt und damit einerseits für ein hohes Untergrundrauschen sorgt und andererseits auch zu unnötiger Strahlenbelastung des untersuchten Patienten führt.

Es ist daher Aufgabe der Erfindung, eine Fokus-Detektor-Anordnung zu finden, welche eine bei ausreichender Dosisleistung für medizinische Zwecke eine verbesserte Dosisnutzung zur Phasenkontrastbildgebung erreicht. Es soll also das Verhältnis von zur Phasenkontrastmessung nutzbaren Strahlung zur Strahlung, die nur zu Absorptionsmessungen nutzbar ist verbessert werden.

Diese Aufgabe wird durch die Merkmale der unabhängigen Patentansprüche gelöst. Vorteilhafte Weiterbildungen der Erfindung sind Gegenstand untergeordneter Ansprüche.

Die Erfinder haben erkannt, dass es möglich ist, den Effekt der Erzeugung eines Bündels von quasi-kohärenten Strahlen mit gitterartigem Ursprung durch ein Quellengitter auch direkt auf einer Anode auszuführen, indem streifenförmig angeordnete Bereiche erzeugt werden, die eine unterschiedliche Strahlungsemission aufweisen. Vorteilhaft kann damit auch eine Bewegung des Quellengitters nachgebildet werden.

Demgemäß schlagen die Erfinder vor, die an sich bekannte Fokus-Detektor-Anordnung einer Röntgenapparatur zur Erzeugung projektiver oder tomographischer Phasenkontrastaufnahmen von einem Untersuchungsobjekt, zumindest bestehend aus:
- einer auf einer ersten Seite des Untersuchungsobjektes angeordneten Strahlungsquelle, welche ein Bündel von kohärenten Strahlen mit gitterartigem Ursprung erzeugt,
- einem auf der gegenüberliegenden zweiten Seite des Untersuchungsobjektes im Strahlengang angeordneten Phasengitter, welches benachbarte kohärente Strahlen zur Beugung bringt und somit ein von der Phasenverschiebung von Teilbereichen des Untersuchungsobjektes abhängiges Interferenzmuster / stehendes Wellenfeld der Röntgenstrahlung in einem bestimmten Energiebereich der Röntgenstrahlung erzeugt, und
- einem Analyse-Detektor-System, welches zumindest das vom Phasengitter erzeugte Interferenzmuster bezüglich seiner örtlichen Intensitätsverteilung zur Bestimmung einer örtlichen Phasenverschiebung detektiert, dahingehend zu verbessern, dass das Bündel von kohärenten Strahlen mit gitterartigem Ursprung durch eine Anode erzeugt wird, welche streifenförmig angeordnete Bereiche mit unterschiedlicher Strahlungsemission aufweist, die parallel zu den Gitterlinien des Phasengitters verlaufen.

In einer ersten Ausführungsvariante der Fokus-Detektor-Anordnung wird vorgeschlagen, dass zumindest die Oberfläche der Anode im Bereich eines zum Betrieb der Röntgenröhre erzeugten Elektronenstrahlbrennfleckes streifenförmig angeordnete Bereiche unterschiedlichen Materials aufweist.

Hierbei können Mittel zur Verschiebung der Anode, vorzugsweise senkrecht zur Streifenlängsrichtung, vorgesehen werden, wobei diese Mittel zur Verschiebung der streifenförmig angeordneten Bereiche, vorzugsweise senkrecht zur Streifenlängsrichtung wirkend, vorgesehen sind.

Alternativ wird vorgeschlagen zwischen Kathode und Anode eine Elektronenmaske mit streifenartigen Öffnungen anzuordnen, welche auf der Anode abgebildet werden und dadurch auf der Anode zu streifenartigen Bereichen unterschiedlicher Strahlungsemission führen. Ergänzend kann zwischen der Elektronenmaske und der Anode und/oder zwischen Kathode und Elektronenmaske mindestens eine elektronenoptische Linse angeordnet werden.

Die elektronenoptische Linse kann als magnetische Feldlinse oder als elektrische Feldlinse ausgebildet werden.

Erfindungsgemäß wird auch vorgeschlagen, dass Mittel zur Verschiebung der Elektronenmaske, vorzugsweise zur Verschiebung senkrecht zur Streifenlängsrichtung, vorgesehen werden.

Außerdem können Mittel zur Variation mindestens einer elektronenoptischen Linse vorgesehen werden, welche eine Verschiebung der Maskenabbildung auf der Anode, vorzugsweise senkrecht zur Streifenlängsrichtung, bewirkt.

In einer weiteren Ausführung wird auch vorgeschlagen, dass die Anode zumindest im Bereich eines zum Betrieb der Röntgenröhre erzeugten Elektronenstrahlbrennfleckes streifenförmig angeordnete Höhen und Tiefen aufweist, wodurch Abschattungen entstehen oder aufgrund der dann sich ausbildenden Feldlinien die Elektronen bevorzugt an den Höhen auf die Anode treffen und dort bevorzugt Röntgenstrahlung produzieren.

Der Verlauf der Höhen und Tiefen kann beispielsweise wellenförmig, vorzugsweise sinusförmig, oder auch sägezahnartig, trapezartig oder rechteckig ausgebildet werden.

Außerdem kann die Anode bevorzugt wegen der besseren Kühlungssituation als Drehanode ausgeführt werden. Hierbei kann die Drehanode je nach Anforderung in Rotationsrichtung ausgerichtete Streifen, Streifen auf einer Kegelmanteloberfläche der Drehanode oder Streifen auf einer Zylindermanteloberfläche der Drehanode aufweisen.

Weiterhin können in einer besonderen Ausführung der Fokus-Detektor-Anordnung die Streifen der Drehanode eine zur Drehachse der Drehanode axiale Richtungskomponente aufweisen und Mittel zur Erzeugung und Steuerung einer stroboskopartigen Pulsation des Röhrenstromes vorgesehen werden. Hierbei können Mittel zur Abstimmung von Frequenz und Phase der Pulsation des Röhrenstromes und der Drehzahl derart vorgesehen sein, dass die Position der Streifen unterschiedlichen Materials im Maximum des Röhrenstroms relativ zur Röntgenröhre unverändert bleibt.

Die Mittel zur Abstimmung von Frequenz und Phase der Pulsation des Röhrenstromes und der Drehzahl können auch derart ausgebildet sein, dass die Position der Streifen unterschiedlichen Materials im Maximum des Röhrenstroms relativ zur Röntgenröhre, vorzugsweise schrittweise, in Rotationsrichtung zur Messung der Phasenverschiebung bei ortsfestem Phasengitter und ortsfestem Analysengitter wandert. Hierdurch wird die an sich bekannte Bewegung des Quellengitters nachgebildet.

Grundsätzlich sollten in dieser Fokus-Detektor-Anordnung die Streifen parallel zu den Gitterlinien des Phasengitters angeordnet sein.

Die Streifen können allerdings auch einen Winkel, vorzugsweise 45°, zur radialen Richtung aufweisen.

In einer grundsätzlich anderen Variante der erfindungsgemäßen Fokus-Detektor-Anordnung schlagen die Erfinder vor, dass die Röntgenröhre Mittel zur Erzeugung und Ablenkung eines gebündelten Elektronenstrahls aufweist, wobei der Elektronenstrahl auf einer Anodenoberfläche entlang mindestens einer gedachten Gitterlinie bewegt wird.

In einer vorteilhaften Weiterbildung können mehrere Gitterlinien vorgesehen sein und der Elektronenstrahl kann von Gitterlinie zu Gitterlinie springen. Hierbei können die Gitterlinien Abstände untereinander aufweisen, die ein ganzzahliges Vielfaches eines Grundabstandes darstellen. Hierdurch wird die Periodizität des Gitters gewahrt, jedoch gleichzeitig unterschiedliche Abstände ermöglicht. Als einfachste Variante dieser Ausführung kann jedoch einfach ein periodisches Gitter angesehen werden, in dem alle Gitterlinien parallel mit gleichem Abstand verlaufen, wobei der Elektronenstrahl die Gitterlinien nacheinander oder in beliebiger Reihenfolge abtastet.

Bevorzugt kann hier eine Drehanode zur besseren Wärmeableitung verwendet werden.

In einer bevorzugten Ausführung kann diese Drehanode eine kegelförmige Anodenfläche aufweisen, wobei die Zeilen auf dieser Oberfläche radial oder tangential zur Drehachse der Drehanode ausgerichtet sind.

In einer weiteren Variante dieser Ausführung mit gebündeltem und gelenktem Elektrodenstrahl kann die Drehanode eine zylinderförmige Anodenfläche aufweisen, wobei die Zeilen auf dieser Oberfläche parallel oder senkrecht zur Drehachse ausgerichtet sind. Auch ist es möglich, die Zeilen schräg zur Drehachse und zur radialen Richtung auszurichten.

Zusätzlich schlagen die Erfinder in dieser Ausführungsvariante vor, dass die Abtastperiode, also die Periode eines Umlaufes, des Elektronenstrahls klein ist (Faktor 1/2 - 1/10), vorzugsweise sehr klein ist (Faktor <1/10), gegen die Abtastperiode des Detektors im Analyse-Detektor-System.

Außerdem können die Mittel zur Ablenkung des Elektronenstrahls derart gestaltet sein, dass die Bewegung eines Quellengitters zur Bestimmung der Phasenverschiebung nachgebildet wird.

Die oben beschriebenen Fokus-Detektor-Anordnungen können ohne abschließende Aufzählung beispielsweise in Röntgen-System zur Erzeugung projektiver Phasenkontrastaufnahmen, in Röntgen-C-Bogen-System zur Erzeugung projektiver oder tomographischer Phasenkontrastaufnahmen oder in Röntgen-CT-System zur Erzeugung tomographischer Phasenkontrastaufnahmen verwendet werden.

Entsprechend dem grundlegenden Erfindungsgedanken schlagen die Erfinder auch ein Verfahren zur Erzeugung projektiver oder tomographischer Röntgen-Phasenkontrastaufnahmen von einem Untersuchungsobjekt mit Hilfe einer Fokus-Detektor-Anordnung, enthaltend eine Röntgen-Strahlungsquelle, ein Phasengitter und ein Analyse-Detektor-System, vor, bei dem ein Bündel von kohärenten Strahlen mit gitterartigem Ursprung durch eine Anode erzeugt wird, welche streifenförmig angeordnete Bereiche mit unterschiedlicher Strahlungsemission aufweist, die parallel zu den Gitterlinien des Phasengitters verlaufen.

Beispielsweise können die Streifen unterschiedlicher Strahlungsemission durch streifenförmig angeordnete Bereiche unterschiedlichen Materials erzeugt werden.

Auch können die Streifen unterschiedlicher Strahlungsemission durch streifenförmig angeordnete Bereiche unterschiedlicher Höhe und Tiefe erzeugt werden.

Es kann eine Drehanode genutzt werden, um eine bessere Wärmeableitung zu bewirken und/oder die Bewegung des ersetzten Quellengitters nachzubilden, wobei vorzugsweise die Streifen der Drehanode mit einer zur Drehachse der Drehanode axialen Richtungskomponente betrieben werden und der Röhrenstrom stroboskopartig gepulst wird. Die Frequenz und Phase der Pulsation des Röhrenstromes auf die Drehfrequenz der Drehanode können dabei also wahlweise derart aufeinander abgestimmt werden, dass die Position der Streifen unterschiedlicher Strahlungsemission im Maximum des Röhrenstroms relativ zur Röntgenröhre unverändert bleibt oder dass die Bewegung eines Quellengitters zur Bestimmung der Phasenverschiebung nachgebildet wird.

Gemäß einer anderen Ausbildung des erfindungsgemäßen Verfahrens schlagen die Erfinder auch vor, dass zur Erzeugung eines Bündels von kohärenten Strahlen ein Elektronenstrahl auf der Anodenoberfläche entsprechend den Gitterlinien eines röntgenoptischen Quellengitters bewegt wird, wobei die Gitterlinien des simulierten Quellengitters ortsfest bleiben.

Außerdem wird auch vorgeschlagen, dass zur Erzeugung eines Bündels von kohärenten Strahlen ein Elektronenstrahl auf der Anodenoberfläche entsprechend den Gitterlinien eines röntgenoptischen Quellengitters bewegt wird, wobei die Bewegung der Gitterlinien des simulierten Quellengitters zur Bestimmung der Phasenverschiebung nachgebildet wird.

Zur Vollständigkeit wird darauf hingewiesen, dass bei allen hier dargestellten Ausführungsvarianten der Verlauf der Intensitätsmaxima bildenden Streifen oder Schlitze parallel tangential oder schräg zur Drehachse ausgerichtet sein kann.

Im Folgenden wird die Erfindung anhand bevorzugter Ausführungsbeispiele mit Hilfe der Figuren näher beschrieben, wobei nur die zum Verständnis der Erfindung notwendigen Merkmale dargestellt sind. Hierbei werden die folgenden Bezugszeichen verwendet: 1: CT-System; 2: erste Röntgenröhre; 3: erster Detektor; 4: zweite Röntgenröhre; 5: zweiter Detektor; 6: Gantrygehäuse; 7: Patient; 8: Patientenliege; 9: Systemachse; 10: Steuer- und Recheneinheit; 11: Speicher; 12: Anodenbasis; 13: Streifen; 14: Elektronenstrahl; 15: Elektronenmaske; 16: Anode; 17.1, 17.2 18.1, 18.2: Plattenelektroden; 19: Einschnitt; 20: Plateau; 21: Verlauf der Quellintensität; d: Abstand des Phasengitters G₁ zum Analysengitter G₂; D₁: Detektor; e⁻: Elektronen; Eᵢ, Eⱼ: Detektorelement; G₁: Phasengitter; G₂: Analysengitter; I(Eₓ(x_{G})): gemessene Intensität am Detektorelement Eₓ beim Gitterversatz x_{G}; I: gemessene Intensität des Photonenflusses; Prgₙ: Programme; Sᵢ: Röntgenstrahlen; x_{G}: Versatz des Analysengitters; ϕ_{×}: Phasenverschiebung am Detektorelement Eₓ; ϕᵢⱼ: relative Phasenverschiebung zwischen den Detektorelementen; λ: Brems- und Röntgenstrahlung.

Es zeigen im Einzelnen:
- FIG 1: einen Längsschnitt durch eine Prinzipdarstellung einer Fokus-Detektor-Anordnung mit Phasengitter, Analysengitter und Detektor zur Darstellung der Interferenzerscheinung;
- FIG 2: einen Intensitätsverlauf an ausgewählten Detektorelementen bei Relativverschiebung eines Gitters;
- FIG 3: eine Ausführung einer Anode mit Materialstreifen aus Material mit unterschiedlichem Z gegenüber dem Anodenbasismaterial;
- FIG 4: eine Ausführung einer Drehanode mit radial ausgerichteten Materialstreifen aus Material mit unterschiedlichem Z gegenüber dem Anodenbasismaterial;
- FIG 5: eine Ausführung einer Anode mit einem Elektrodenstrahl, der durch eine Elektrodenmaske selektiv gerichtet ist;
- FIG 6: eine Ausführung einer Anode mit gebündeltem Elektronenstrahl und einer gesteuerten Strahlablenkungsvorrichtung;
- FIG 7: eine Ausführungsvariante einer Anode mit Einschnitten;
- FIG 8: Ausführungsvarianten von verschiedenen Einschnitten in das Anodenmaterial im Querschnitt; und
- FIG 9:: ein Röntgen-CT-System in 3D-Ansicht mit erfindungsgemäßem Fokus-Detektor-System.

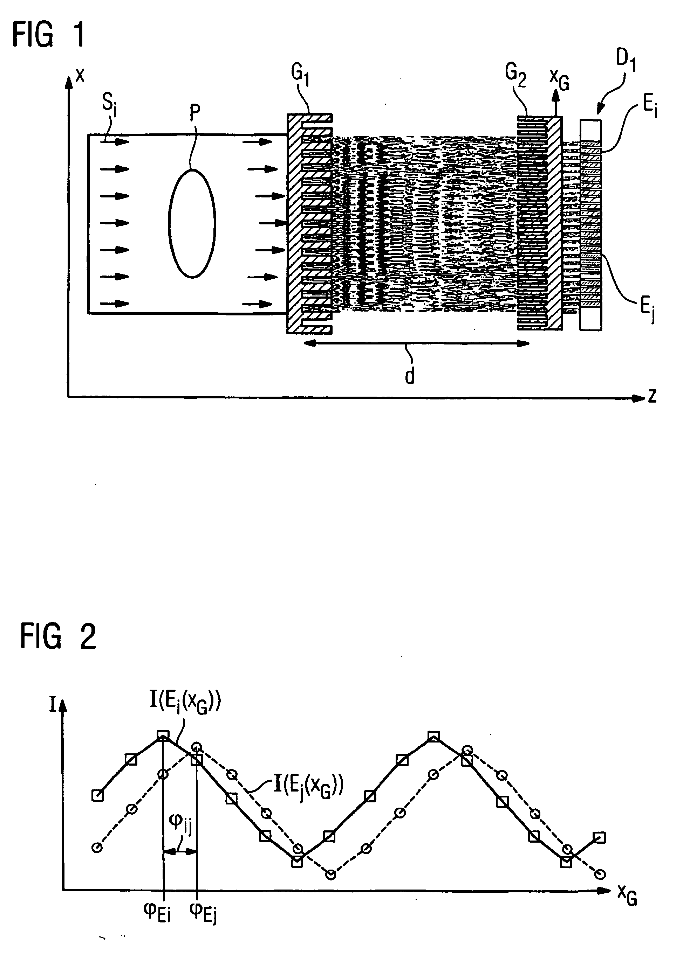

Zum besseren Verständnis wird nachfolgend das grundsätzliche Prinzip der Phasenkontrastmessung mit den Figuren 1 bis 2 beschrieben.

Die **Figur 1** zeigt eine vom Fokus kommende quasi-kohärente Strahlung oder von einem Quellengitter kommende individuell kohärente Strahlung, die eine Probe P durchdringt, wobei es nach dem Durchdringen der Probe P zu Phasenverschiebungserscheinungen kommt. Hierdurch wird beim Durchtritt durch das Gitter G₁ ein Interferenzmuster, welches durch die Grauschattierung dargestellt ist, erzeugt, das mit Hilfe des Gitters G₂ auf den anschließenden Detektor D₁ und dessen Detektorelementen zu unterschiedlichen Strahlungsintensitäten je Detektorelement führt, wobei sich in einem so genannten Talbotabstand ein Interferenzmuster, ein so genanntes Moire-Muster, ausbildet. Betrachtet man beispielsweise das Detektorelement Eᵢ in Abhängigkeit eines Versatzes x_{G} des Analysengitters G₂ und trägt die Intensität I (Eᵢ(X_{G})) als Funktion des Versatzes x_{G} über die Intensität I, so erhält man einen, in der **Figur 2** gezeigten, sinusförmigen An- und Abstieg der Intensität I an diesem Detektorelement Eᵢ. Trägt man diese gemessenen Strahlungsintensitäten I für jedes Detektorelement Eᵢ beziehungsweise Eⱼ in Abhängigkeit vom Versatz x_{G} auf, so erlässt sich für die verschiedenen Detektorelemente, die ja letztendlich den räumlichen Röntgenstrahl zwischen dem Fokus und dem jeweiligen Detektorelement bilden die Funktion I(Eᵢ(x_{G})) beziehungsweise I (Eⱼ(x_{G})) annähern. Aus den Funktionen lässt sich für jedes Detektorelement die Phasenverschiebung ϕ und die relative Phasenverschiebung ϕᵢⱼ zwischen den Detektorelementen bestimmen.

Es lässt sich somit für jeden Strahl im Raum durch mindestens drei Messungen mit jeweils versetztem Analysengitter die Phasenverschiebung je Strahl ermitteln, woraus entweder im Fall von projektiven Röntgenaufnahmen direkt die Pixelwerte einer projektiven Aufnahme berechnet werden können oder es werden im Fall einer CT-Untersuchung Projektionen erstellt, deren Pixelwerte der Phasenverschiebung entsprechen, so dass hieraus mit Hilfe an sich bekannter Rekonstruktionsmethoden berechnet werden kann, welches Volumenelement im Untersuchungsobjekt welchen Anteil an der gemessenen Phasenverschiebung zuzusprechen ist. Hieraus errechnen sich damit Schnittbilder oder Volumendaten, die die örtliche Wirkung des untersuchten Objektes bezüglich der Phasenverschiebung einer Röntgenstrahlung widerspiegelt. Da bereits geringe Unterschiede in der Zusammensetzung einen starken Effekt auf die Phasenverschiebung ausüben, lassen sich hierdurch sehr detailreiche und kontraststarke Volumendaten von an sich relativ ähnlichen Materialien, insbesondere von Weichteilgewebe, wiedergeben.

Diese Variante der Detektion von Phasenverschiebungen der Röntgenstrahlen, die ein Untersuchungsobjekt durchdringen, mit Hilfe eines mehrfach versetzten Analysengitters und Messung der Strahlungsintensität auf einem Detektorelement hinter dem Analysengitter bedingt, dass von jedem Röntgenstrahl mindestens drei Messungen bei jeweils verschobenem Analysengitter durchgeführt werden müssen.

Grundsätzlich besteht auch die Möglichkeit, auf ein derartiges Analysengitter zu verzichten und stattdessen einen ausreichend fein strukturierten Detektor zu verwenden, wobei in diesem Fall weniger Dosisverluste bei der Messung auftreten und mit einer einzigen Messung die Phasenverschiebung im betrachteten Strahl bestimmt werden kann.

Zur Messung des Phasenkontrastes ist es notwendig, kohärente Strahlung zu verwenden. Erfindungsgemäß wird hierzu ein Feld (Array) individuell kohärenter Strahlung nicht durch ein Quellengitter hinter einem flächig ausgebildeten Fokus, sondern durch eine gitterartige Ausgestaltung des Elektronenstrahlbrennflecks auf der Anode zur Nachbildung eines solchen Gitters erzeugt.

Dieses Array individuell kohärenter, aber zueinander inkohärenter Quellen kann hergestellt werden, indem eine entsprechende Intensitätsverteilung der von der Röntgenröhre emittierten Röntgenstrahlen erzeugt wird. Dies kann auf die verschiedene Weise erreicht werden:

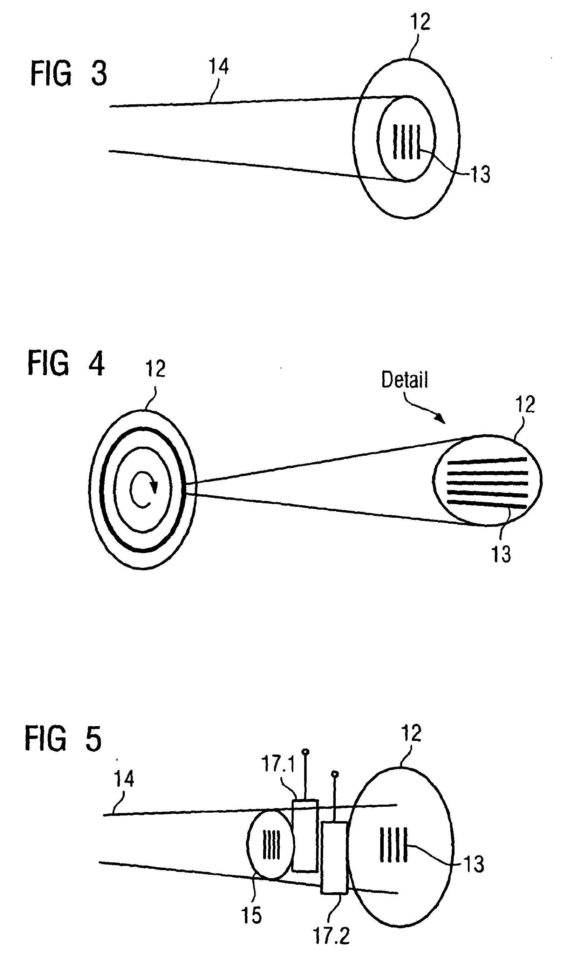

Eine erste mögliche Ausführungsform ist in der **Figur 3** gezeigt, in der ein relativ weiter Elektronenstrahl 14 auf eine Anodenbasisplatte 12 gerichtet ist. Die Anodenbasisplatte 12 besteht aus einem Material mit niedrigem Z-Wert, welches bevorzugt hohe Wärmeleitfähigkeit, hohen Schmelzpunkt, gute Stabilität und ausreichende Stromleitfähigkeit aufweisen sollte. Beispielsweise kann hier Aluminium, Beryllium oder Diamant verwendet werden. Bei Diamant kann die Stromleitfähigkeit beispielsweise dadurch erzielt werden, dass das Material dotiert oder mit einer leitenden Schicht beschichtet wird. An den streifenförmigen Bereichen 13, wo Röntgenstrahlen bevorzugt emittiert werden sollen, soll ein Material mit hohem Z-Wert, z.B. Kupfer, Molybdän oder Wolfram, vorliegen. Die aus einem Material mit hohem Z-Wert hergestellten Streifen 13 emittieren folglich Röntgenstrahlen mit relativ hoher Intensität. Durch eine geeignete Wahl des Materials entsprechend der vorliegenden Beschleunigungsspannung können insbesondere die charakteristischen Linien des Materials emittiert werden, während das umgebende Material vorzugsweise keine charakteristischen Linien in diesem Bereich aufweist. Es ist zwar anzumerken, dass auch dieses Material charakteristische Röntgenstrahlung emittiert, jedoch liegt deren Energie relativ niedrig und wird weitgehend bereits durch das Röhrenfenster absorbiert. Außerdem ist die Effektivität der Erzeugung von Bremsstrahlung geringer, da diese proportional zum Z-Wert ist. Somit werden in Bereichen, in denen die Anodenbasisplatte von dem Elektronenstrahl getroffen wird, zwar ebenfalls Röntgenstrahlen erzeugt, jedoch insgesamt mit wesentlich geringerer Intensität als in den streifenförmigen Bereichen mit hohem Z-Wert.

Vorteilhaft kann auch eine Röntgenröhre mit rotierender Anode verwendet werden, die eine erhöhte Wärmekapazität für einen höheren Röntgenstrahlenfluss liefert. In diesem Fall können viele radial orientierte Streifen entlang einer ganzen kreisförmigen Bahn verteilt werden. Ein Beispiel hierfür ist in der **Figur 4** dargestellt.

Bei kontinuierlicher Röntgenstrahlenemission verschieben sich jedoch bei dieser Anordnung die elementaren Quellen wegen der Anodenrotation ständig bezüglich des Interferometergitters. Dieser Effekt kann einerseits genutzt werden, um ein bewegtes Quellengitter zur Phasenbestimmung zu simulieren, andererseits kann dieser Effekt allerdings auch dadurch vermieden werden, indem der Elektronenstrahl und damit auch die Röntgenstrahlenemission synchron zur Anodenrotation derart gepulst wird, dass deren Maximum immer dann erreicht wird, wenn die Streifen um eine Periode weitergewandert sind. Durch einen stroboskopischen Effekt erscheint die Position der elementaren Röntgenstrahlenemitter dann bei einer Betrachtung vom Detektor aus statisch zu sein.

Durch Einstellen der Phase zwischen Pulsierung und Rotation kann zudem eine für die Phasenzerlegung erforderliche Quellenverschiebung, die sich von der Rotationsgeschwindigkeit der Anode unterscheidet, implementiert werden.

In einer weiteren Ausführungsvariante wird vorgeschlagen, einen Teil des auf die Anodenplatte 16 auftreffenden Elektronenstrahls 14 unter Verwendung einer Elektronenmaske 15, wie es in der **Figur 5** skizziert ist, zu beschneiden. Die Elektronenmaske 15 kann mit einem bestimmten Potenzial (Spannung) verbunden werden. Diese Spannung sollte dabei niedrig genug sein, um zu vermeiden, dass die auftreffenden Elektronen bereits eine zu hohe kinetische Energie erreichen, wodurch die Temperatur der Elektronenmaske 15 zu stark ansteigen würde und zusätzlich unerwünschte sekundäre Röntgenstrahlung erzeugt werden würde. Dies kann vermieden werden, indem zum Beispiel die Elektronenmaske mit einer Spannung unterhalb der Energie, auf die der Interferometeraufbau eingestellt ist, beaufschlagt wird.

Zusätzlich kann hierdurch auf vorteilhafte Weise diese Elektronenmaske als Fokussierungselektrode eingesetzt werden, welche die erzeugten Elektronen auf die Anodenoberfläche fokussiert. Dazu kann die Maske auch an eine wohl definierte Steuerspannung (Fokussierspannung) angeschlossen werden. Bei dieser verbesserten Anordnung entfernt die Elektronenmaske keine Elektronen, sondern konzentriert den austretenden Elektronenstrahl in mehrere stark fokussierte Teilstrahlen. Hierdurch wird die Effizienz stark verbessert.

Gemäß einer weitergehenden anderen beziehungsweise ergänzenden Ausführungsform der Fokus-Detektor-Anordnung kann der auf der Anode auftreffende Elektronenstrahl unter Verwendung eines elektrischen Feldes - erzeugt durch die optionalen Elektrodenplatten 17.1 und 17.2 - oder eines Magnetfelds, also einer Elektronenoptik, entsprechend abgelenkt werden. Zudem kann durch eine solche Anordnung der Elektronenstrahl ein- und ausgeschaltet werden.

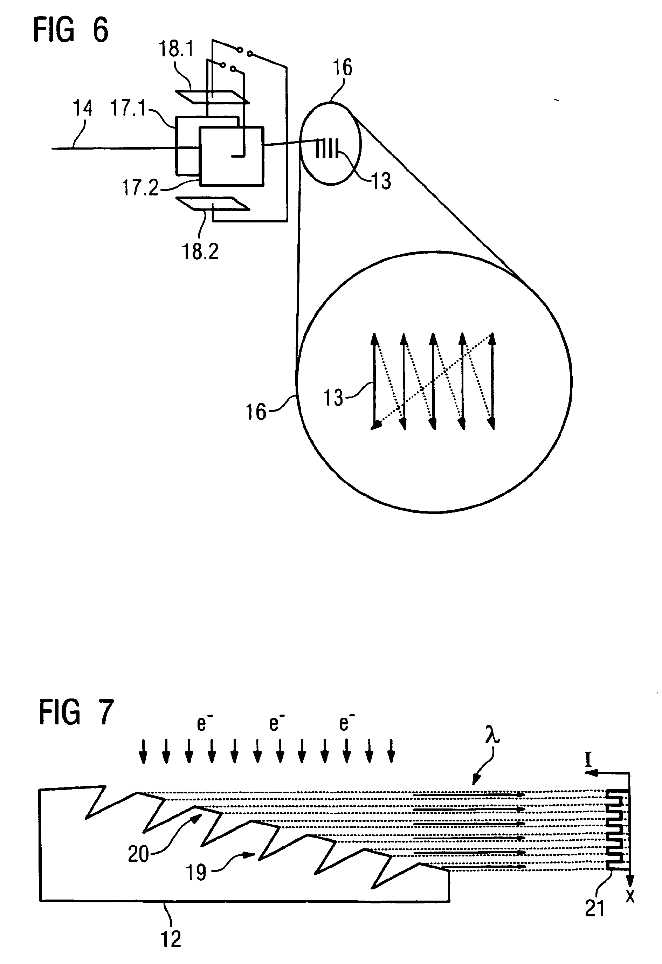

Beispielhaft und stark schematisiert ist eine solche Anordnung in der **Figur 6** für den Fall einer elektrostatischen Optik gezeigt. In dieser Figur ist ein gebündelter Elektronenstrahl 14 dargestellt, der durch zwei senkrecht zueinander wirkende Plattenelektrodenpaare 17.1, 17.2 und 18.1, 18.2 bezüglich seiner Auslenkung in seiner Richtung gesteuert wird. Durch eine entsprechende Steuerung der Plattenelektrodenpaare kann der Elektronenstrahl ähnlich der Abtastung eines Fernsehbildes zeilenartig mit dem gewünschten Abstand der Zeilen die Anode "abtasten" und dabei die erwünschte Röntgenstrahlung erzeugen. Grundsätzlich wird hierbei, betrachten man eine Momentaufnahme, lediglich ein Punktfokus erzeugt, allerdings wird über längere Strahlungszeit hinweg gemittelt ein Streifenmuster, das aus zumindest einem oder mehreren Streifen entsprechend den Linien eines Quellgitters besteht, erzeugt. Im zeitlichen Mittel wird also auch hierdurch die Funktion eines Quellengitters erreicht.

Bezüglich der Pulsation des Elektronenstrahls ist zu bemerken, dass diese beispielsweise durch eine gezielte periodische Veränderung der Kathodentemperatur, schnelle und starke Ablenkung des Elektronenstrahls, Feldemissionskathoden, elektrisch steuerbare Elektronenemission, Photoemissionskathoden, Kathoden mit licht- oder laserinduzierter Emission, Streak-Röhren, gegatete Elektronenröhren wie eine Triode oder Pentode oder auch durch Wanderfeldröhren geschehen kann.

Alternativ kann ein linienförmiger Fokus und nur eine einzelne Achsenablenkung senkrecht hierzu angewendet werden. Bei diesem Ansatz wird die Hot-Spot-Temperatur entlang des linienförmigen Fokus verteilt. Wie bereits erwähnt kann man auch hier eine für die Phasenzerlegung mögliche, beziehungsweise falls keine beweglichen Gitter oder Detektoren eingesetzt werden, erforderliche Quellenverschiebung über das Horizontalablenkmittel erhalten.

Eine weitere Verbesserungsmöglichkeit besteht in der Verwendung einer Röntgenröhre mit rotierender Anode, wahlweise mit Elektronenstrahlablenkung. Bei dieser Anordnung wird die Hot-Spot-Temperatur entlang einer längeren kreisförmigen Spur verteilt. Die Rotation mit ausreichender Geschwindigkeit verschmiert die thermische Belastung homogen über die Bahn des Elektronenstrahls.

Es ist weiterhin zu bemerken, dass die maximale Brillanz einer Röntgenröhre durch die Ableitung der beim Brennpunkt erzeugten Wärme begrenzt wird. Wenn eine aus mehreren Streifen bestehende Röntgenquelle, entsprechend den oben dargestellten erfindungsgemäßen Ausführungsvarianten, verwendet wird, so ergibt sich auch eine verbesserte Wärmeableitung auf der Anodenoberfläche. Im Gegensatz zu einer Anordnung, die aus einem flächigen Brennpunkt und Quellengitter besteht, wird an den Bereichen zwischen den Streifen keine oder weniger Wärme produziert, so dass deshalb eine höhere Brillanz der Strahlung erreicht werden kann.

Bezüglich der zuvor beschriebenen Anordnung mit einer Anode aus unterschiedlichen Materialien, die streifenförmig angeordnet sind, ergibt sich die Situation, dass zwar bei den Streifen und in den Bereichen zwischen den Streifen fast der gleiche Wärmefluss erzeugt wird, jedoch das Material zwischen den Streifen einen niedrigeren Z-Wert aufweist und damit eine signifikant höhere Eindringtiefe der Elektronen vorliegt, so dass der Wärmefluss auch tiefer reicht und damit eine verbesserte Wärmeableitung vorliegt.

Vorteilhaft ist auch, dass zur "virtuellen" Bewegung des "Gitters" keine mechanischen Vorrichtungen notwendig sind, sondern diese auf einfache Weise und sehr präzise, schnell und ohne mechanischen Verschleiß elektronisch erzeugt werden kann.

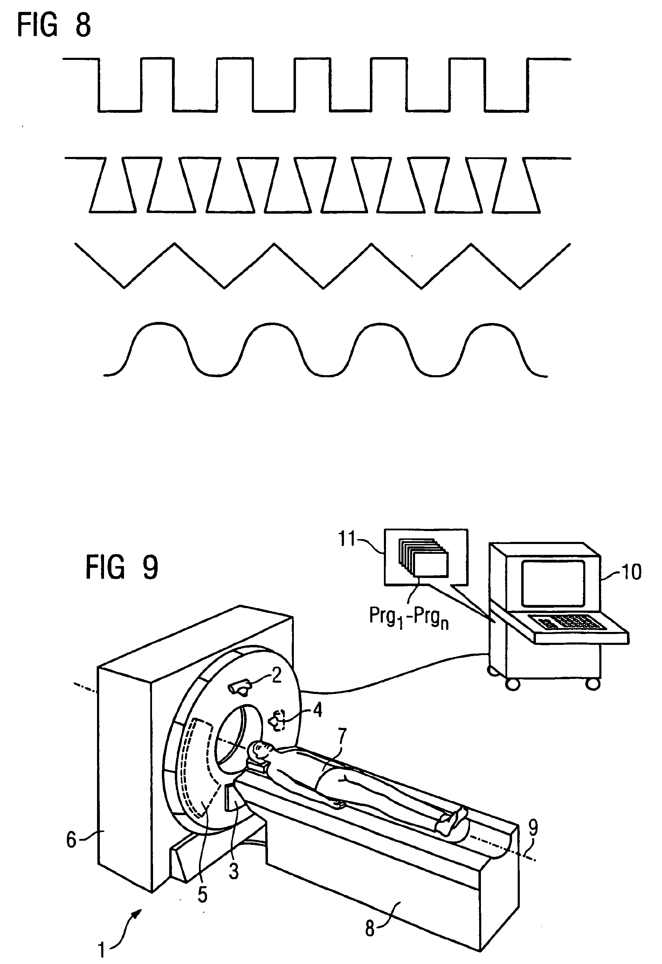

Eine andere Ausführungsvariante einer erfindungsgemäßen Anode 12 ist in der **Figur 7** dargestellt. Diese weist Einschnitte 19 auf, welche das Anodenmaterial gegenüber den anfliegenden Elektronen e⁻ abschatten, während auf den Plateaus 20 der Anode die Elektronen e⁻ vermehrt auftreffen. Entsprechend entstehen auf der Anodenoberfläche streifenförmige Bereiche mit vermehrter und verminderter Quellintensität an erzeugter Brems- und Röntgenstrahlung λ. Rechts in der Figur ist die Quellintensität Q der Röntgenstrahlung gegenüber einer gewillkürten x-Achse schematisch als Treppenlinie 21 aufgetragen. Es wird darauf hingewiesen, dass auch andere Ausführungsvarianten, z.B. nutenförmige Vertiefungen oder auch ein wellen- oder sinusförmiger Oberflächenverlauf, möglich sind. Wesentlich ist hierbei lediglich, dass auf der Anodenoberfläche Röntgenstrahlung mit unterschiedlicher Intensität entsteht. Vier Beispiele für andere mögliche Oberflächenverläufe sind in der **Figur 8** im Querschnitt gezeigt.

Ein vollständiges Computer-CT-System zur Verwendung des erfindungsgemäßen Fokus-Detektor-Systems beziehungsweise Durchführung des erfindungsgemäßen Verfahrens ist in der **Figur 9** dargestellt. Diese zeigt das CT-System 1, welches über ein erstes Fokus-Detektor-System mit einer Röntgenröhre 2 und einem gegenüberliegenden Detektor 3 verfügt, die auf einer nicht näher dargestellten Gantry in einem Gantrygehäuse 6 angeordnet sind. Die Röntgenröhre 2 enthält hierbei einen erfindungsgemäßen Multistreifenfokus, welcher quasi-kohärente Röntgenstrahlen erzeugt. Im Strahlengang des ersten Fokus-Detektor-Systems ist weiterhin ein röntgenoptisches Gittersystem, wie es beispielsweise in der Figur 1 gezeigt ist, angeordnet, so dass der Patient 7, der sich auf einer längs der Systemachse 9 verschiebbaren Patientenliege 8 befindet, in den Strahlengang des ersten Fokus-Detektor-Systems geschoben werden kann und dort abgetastet wird. Hierbei wird die Phasenverschiebung der durchtretenden Röntgenstrahlen gemessen und über an sich bekannte Rekonstruktionsverfahren die räumliche Verteilung der Brechungsindizes bestimmt. Die Steuerung des CT-Systems wird durch eine Rechen- und Steuereinheit 10 durchgeführt, in der in einem Speicher 11 Programme Prg₁ bis Prgₙ gespeichert sind, die im Betrieb das zuvor beschriebene Verfahren durchführen und auch die erfindungsgemäße Röntgenröhre mit ihrem Multistreifenfokus steuern und aus den gemessenen strahlenabhängigen Phasenverschiebungen entsprechende tomographische Bilder rekonstruieren.

Optional kann anstelle des einzigen Fokus-Detektor-Systems ein zweites Fokus-Detektor-System im Gantrygehäuse angeordnet werden. Dieses ist in der Figur 9 durch die gestrichelt gezeigte Röntgenröhre 4 und den gestrichelt dargestellten Detektor 5 angedeutet.

Ergänzend ist noch darauf hinzuweisen, dass mit den gezeigten Fokus-Detektor-Systemen nicht nur Phasenverschiebungen der Röntgenstrahlung gemessen werden können, sondern diese weiterhin auch zur konventionellen Messung der Strahlungsabsorption und Rekonstruktion von entsprechenden Absorptionsaufnahmen geeignet sind. Gegebenenfalls können auch kombinierte Absorptions- und Phasenkontrastaufnahmen erzeugt werden.

Es versteht sich, dass die vorstehend genannten Merkmale der Erfindung nicht nur in der jeweils angegebenen Kombination, sondern auch in anderen Kombinationen oder in Alleinstellung verwendbar sind, ohne den Rahmen der Erfindung zu verlassen.

## Patentansprüche

1. Fokus-Detektor-Anordnung einer Röntgenapparatur (1) zur Erzeugung projektiver oder tomographischer Phasenkontrastaufnahmen von einem Untersuchungsobjekt (7, P), zumindest bestehend aus:
einer auf einer ersten Seite des Untersuchungsobjektes (7, P) angeordneten Strahlungsquelle (2), welche ein Bündel von kohärenten Strahlen (Sᵢ) mit gitterartigem Ursprung erzeugt,
einem auf der gegenüberliegenden zweiten Seite des Untersuchungsobjektes im Strahlengang angeordnetes Phasengitter (G₁), welches benachbarte kohärente Strahlen zur Beugung bringt und somit ein von der Phasenverschiebung von Teilbereichen des Untersuchungsobjektes (7, P) abhängiges Interferenzmuster der Röntgenstrahlung in einem bestimmten Energiebereich der Röntgenstrahlung erzeugt, und
einem Analyse-Detektor-System (G₂, D₁), welches zumindest das vom Phasengitter (G₁) erzeugte Interferenzmuster bezüglich seiner örtlichen Intensitätsverteilung (I) zur Bestimmung einer örtlichen Phasenverschiebung (ϕ) detektiert,
**gekennzeichnet durch** eine Anode (12), die das Bündel von kohärenten Strahlen (Sᵢ) mit gitterartigem Ursprung erzeugt und streifenförmig angeordnete Bereiche (13) mit unterschiedlicher Strahlungsemission aufweist, die parallel zu den Gitterlinien des Phasengitters (G₁) verlaufen.

2. Fokus-Detektor-Anordnung gemäß dem voranstehenden Patentanspruch 1, **dadurch gekennzeichnet, dass** zumindest die Oberfläche der Anode (12) im Bereich eines zum Betrieb der Strahlungsquelle (2) erzeugten Elektronenstrahlbrennfleckes streifenförmig angeordnete Bereiche unterschiedlichen Materials aufweist.

3. Fokus-Detektor-Anordnung gemäß dem voranstehenden Patentanspruch 2, **dadurch gekennzeichnet, dass** Mittel (17.1, 17.2, 18.1, 18.2) zur Verschiebung der Anode (12), vorzugsweise senkrecht zur Streifenlängsrichtung, vorgesehen sind.

4. Fokus-Detektor-Anordnung gemäß dem voranstehenden Patentanspruch 2, **dadurch gekennzeichnet, dass** Mittel zur Verschiebung der streifenförmig angeordneten Bereiche unterschiedlichen Materials auf der Anode, vorzugsweise senkrecht zur Streifenlängsrichtung, vorgesehen sind.

5. Fokus-Detektor-Anordnung gemäß einem der voranstehenden Patentansprüche 1 bis 4, **dadurch gekennzeichnet, dass** zwischen Kathode und Anode (12) eine Elektronenmaske (15) mit streifenartigen Öffnungen angeordnet ist, welche auf der Anode (12) abgebildet werden und dadurch auf der Anode (12) zu streifenartigen Bereichen (13) unterschiedlicher Strahlungsemission führen.

6. Fokus-Detektor-Anordnung gemäß dem voranstehenden Patentanspruch 5, **dadurch gekennzeichnet, dass** zwischen der Elektronenmaske (15) und der Anode (12) mindestens eine elektronenoptische Linse (17.1, 17.2) angeordnet ist.

7. Fokus-Detektor-Anordnung gemäß einem der voranstehenden Patentansprüche 5 bis 6, **dadurch gekennzeichnet, dass** zwischen Kathode und Elektronenmaske (15) mindestens eine elektronenoptische Linse angeordnet ist.

8. Fokus-Detektor-Anordnung gemäß einem der voranstehenden Patentansprüche 6 bis 7, **dadurch gekennzeichnet, dass** mindestens eine elektronenoptische Linse eine magnetische Feldlinse ist.

9. Fokus-Detektor-Anordnung gemäß einem der voranstehenden Patentansprüche 6 bis 7, **dadurch gekennzeichnet, dass** mindestens eine elektronenoptische Linse eine elektrische Feldlinse (17.1, 17.2) ist.

10. Fokus-Detektor-Anordnung gemäß einem der voranstehenden Patentansprüche 5 bis 9, **dadurch gekennzeichnet, dass** Mittel zur Verschiebung der Elektronenmaske, vorzugsweise senkrecht zur Streifenlängsrichtung, vorgesehen sind.

11. Fokus-Detektor-Anordnung gemäß einem der voranstehenden Patentansprüche 5 bis 10, **dadurch gekennzeichnet, dass** Mittel zur Variation mindestens einer elektronenoptischen Linse vorgesehen sind, welche eine Verschiebung der Maskenabbildung auf der Anode (12), vorzugsweise senkrecht zur Streifenlängsrichtung, bewirkt.

12. Fokus-Detektor-Anordnung gemäß einem der voranstehenden Patentansprüche 1 bis 11, **dadurch gekennzeichnet, dass** die Anode (12) zumindest im Bereich eines zum Betrieb der Strahlungsquelle (2) erzeugten Elektronenstrahlbrennfleckes streifenförmig angeordnete Höhen (20) und Tiefen (19) aufweist.

13. Fokus-Detektor-Anordnung gemäß dem voranstehenden Patentanspruch 12, **dadurch gekennzeichnet, dass** der Verlauf der Höhen (20) und Tiefen (19) wellenförmig, vorzugsweise sinusförmig, ausgebildet ist.

14. Fokus-Detektor-Anordnung gemäß dem voranstehenden Patentanspruch 12, **dadurch gekennzeichnet, dass** der Verlauf der Höhen (20) und Tiefen (19) sägezahnartig ausgebildet ist.

15. Fokus-Detektor-Anordnung gemäß dem voranstehenden Patentanspruch 12, **dadurch gekennzeichnet, dass** der Verlauf der Höhen (20) und Tiefen (19) trapezartig ausgebildet ist.

16. Fokus-Detektor-Anordnung gemäß dem voranstehenden Patentanspruch 12, **dadurch gekennzeichnet, dass** der Verlauf der Höhen (20) und Tiefen (19) rechteckig ausgebildet ist.

17. Fokus-Detektor-Anordnung gemäß einem der voranstehenden Patentansprüche 2 bis 16, **dadurch gekennzeichnet, dass** die Anode (12) als Drehanode ausgebildet ist.

18. Fokus-Detektor-Anordnung gemäß einem der voranstehenden Patentansprüche 2 bis 16, **dadurch gekennzeichnet, dass** die Drehanode in Rotationsrichtung ausgerichtete Streifen aufweist.

19. Fokus-Detektor-Anordnung gemäß dem voranstehenden Patentanspruch 18, **dadurch gekennzeichnet, dass** die Streifen auf einer Kegelmanteloberfläche der Drehanode angeordnet sind.

20. Fokus-Detektor-Anordnung gemäß dem voranstehenden Patentanspruch 18, **dadurch gekennzeichnet, dass** die Streifen auf einer Zylindermanteloberfläche der Drehanode angeordnet sind.

21. Fokus-Detektor-Anordnung gemäß einem der voranstehenden Patentansprüche 18 bis 20, **dadurch gekennzeichnet, dass**:
die Streifen der Drehanode eine zur Drehachse der Drehanode axiale Richtungskomponente aufweisen und
Mittel zur Erzeugung und Steuerung einer stroboskopartigen Pulsation des Röhrenstromes vorgesehen sind.

22. Fokus-Detektor-Anordnung gemäß dem voranstehenden Patentanspruch 21, **dadurch gekennzeichnet, dass** Mittel zur Abstimmung von Frequenz und Phase der Pulsation des Röhrenstromes und der Drehzahl derart vorgesehen sind, dass die Position der Streifen unterschiedlichen Materials im Maximum des Röhrenstroms relativ zur Strahlungsquelle unverändert bleibt.

23. Fokus-Detektor-Anordnung gemäß dem voranstehenden Patentanspruch 21, **dadurch gekennzeichnet, dass** Mittel zur Abstimmung von Frequenz und Phase der Pulsation des Röhrenstromes und der Drehzahl derart vorgesehen sind, dass die Position der vom Elektronenstrahl beleuchteten Streifen unterschiedlichen Materials im Maximum des Röhrenstroms relativ zur Strahlungsquelle, vorzugsweise schrittweise, in Rotationsrichtung zur Messung der Phasenverschiebung bei ortsfestem Phasengitter und ortsfestem Analysengitter wandert.

24. Fokus-Detektor-Anordnung gemäß einem der voranstehenden Patentansprüche 21 bis 23, **dadurch gekennzeichnet, dass** die Streifen (13) parallel zu den Gitterlinien des Phasengitters angeordnet sind.

25. Fokus-Detektor-Anordnung gemäß einem der voranstehenden Patentansprüche 17 oder 19 bis 24, **dadurch gekennzeichnet, dass** die Streifen (13) in einem Winkel, vorzugsweise 45°, zur radialen Richtung angeordnet sind.

26. Fokus-Detektor-Anordnung gemäß dem voranstehenden Patentanspruch 1, **dadurch gekennzeichnet, dass** die Strahlungsquelle (2) Mittel zur Erzeugung und Ablenkung eines gebündelten Elektronenstrahls aufweist, wobei der Elektronenstrahl auf einer Anodenoberfläche entlang mindestens einer gedachten Gitterlinie bewegt wird.

27. Fokus-Detektor-Anordnung gemäß dem voranstehenden Patentanspruch 26, **dadurch gekennzeichnet, dass** mehrere Gitterlinien vorgesehen sind und der Elektronenstrahl von Gitterlinie zu Gitterlinie springt.

28. Fokus-Detektor-Anordnung gemäß einem der voranstehenden Patentansprüche 26 bis 27, **dadurch gekennzeichnet, dass** die Gitterlinien Abstände untereinander aufweisen, die ganzzahlige Vielfache eines Grundabstandes sind.

29. Fokus-Detektor-Anordnung gemäß einem der voranstehenden Patentansprüche 26 bis 28, **dadurch gekennzeichnet, dass** eine Drehanode vorgesehen ist.

30. Fokus-Detektor-Anordnung gemäß dem voranstehenden Patentanspruch 29, **dadurch gekennzeichnet, dass** die Drehanode eine kegelförmige Anodenfläche aufweist und die Zeilen auf dieser Oberfläche radial zur Drehachse der Drehanode ausgerichtet sind.

31. Fokus-Detektor-Anordnung gemäß dem voranstehenden Patentanspruch 29, **dadurch gekennzeichnet, dass** die Drehanode eine kegelförmige Anodenfläche aufweist und die Zeilen auf dieser Oberfläche tangential zur Drehachse der Drehanode ausgerichtet sind.

32. Fokus-Detektor-Anordnung gemäß dem voranstehenden Patentanspruch 29, **dadurch gekennzeichnet, dass** die Drehanode eine zylinderförmige Anodenfläche aufweist und die Zeilen auf dieser Oberfläche parallel zur Drehachse ausgerichtet sind.

33. Fokus-Detektor-Anordnung gemäß dem voranstehenden Patentanspruch 29, **dadurch gekennzeichnet, dass** die Drehanode eine zylinderförmige Anodenfläche aufweist und die Zeilen auf dieser Oberfläche senkrecht zur Drehachse ausgerichtet sind.

34. Fokus-Detektor-Anordnung gemäß einem der voranstehenden Patentansprüche 30 bis 33, **dadurch gekennzeichnet, dass** die Zeilen schräg zur Drehachse und zur radialen Richtung ausgerichtet sind.

35. Fokus-Detektor-Anordnung gemäß einem der voranstehenden Patentansprüche 26 bis 34, **dadurch gekennzeichnet, dass** die Periode eines Umlaufs des Elektronenstrahls klein mit Faktor 1/2 - 1/10 ist, vorzugsweise sehr klein mit Faktor <1/10 ist, gegen die Abtastperiode des Detektors im Analyse-Detektor-System.

36. Fokus-Detektor-Anordnung gemäß einem der voranstehenden Patentansprüche 26 bis 35, **dadurch gekennzeichnet, dass** die Mittel (17.1, 17.2, 18.1, 18.2) zur Ablenkung des Elektronenstrahls, derart gestaltet sind, dass die Bewegung eines Quellengitters zur Bestimmung der Phasenverschiebung nachgebildet wird.

37. Röntgen-System zur Erzeugung projektiver Phasenkontrastaufnahmen, **dadurch gekennzeichnet, dass** es eine Fokus-Detektor-Anordnung gemäß einem der voranstehenden Patentansprüche 1 bis 36 aufweist.

38. Röntgen-C-Bogen-System zur Erzeugung projektiver oder tomographischer Phasenkontrastaufnahmen, **dadurch gekennzeichnet, dass** es eine Fokus-Detektor-Anordnung gemäß einem der voranstehenden Patentansprüche 1 bis 36 aufweist.

39. Röntgen-CT-System zur Erzeugung tomographischer Phasenkontrastaufnahmen, **dadurch gekennzeichnet, dass** es eine Fokus-Detektor-Anordnung gemäß einem der voranstehenden Patentansprüche 1 bis 36 aufweist.

40. Verfahren zur Erzeugung projektiver oder tomographischer Röntgen-Phasenkontrastaufnahmen von einem Untersuchungsobjekt mit Hilfe einer Fokus-Detektor-Anordnung gemäß einem der Patentansprüche 1 bis 32, enthaltend die Strahlungsquelle (2), das Phasengitter (G₁), das Analyse-Detektor-System (G₂, D₁) und die Anode (12), wobei das Bündel von kohärenten Strahlen mit gitterartigem Ursprung durch die Anode (12) erzeugt wird, welche streifenförmig angeordnete Bereiche mit unterschiedlicher Strahlungsemission aufweist, die parallel zu den Gitterlinien des Phasengitters (G₁) verlaufen.

41. Verfahren gemäß dem voranstehenden Patentanspruch 40, **dadurch gekennzeichnet, dass** die Streifen (13) unterschiedlicher Strahlungsemission durch streifenförmig angeordnete Bereiche unterschiedlichen Materials erzeugt werden.

42. Verfahren gemäß einem der voranstehenden Patentansprüche 40 bis 41, **dadurch gekennzeichnet, dass** die Streifen (13) unterschiedlicher Strahlungsemission durch streifenförmig angeordnete Bereiche unterschiedlicher Höhe (20) und Tiefe (19) erzeugt werden.

43. Verfahren gemäß einem der voranstehenden Patentansprüche 40 bis 41, **dadurch gekennzeichnet, dass** eine Drehanode verwendet wird.

44. Verfahren gemäß dem voranstehenden Patentanspruch 43, **dadurch gekennzeichnet, dass**:
die Streifen der Drehanode mit einer zur Drehachse der Drehanode axialen Richtungskomponente betrieben werden und
der Röhrenstrom stroboskopartig gepulst wird.

45. Verfahren gemäß dem voranstehenden Patentanspruch 44, **dadurch gekennzeichnet, dass** die Frequenz und Phase der Pulsation des Röhrenstromes auf die Dreh frequenz der Drehanode derart abgestimmt wird, dass die Position der Streifen unterschiedlicher Strahlungsemission im Maximum des Röhrenstroms relativ zur Strahlungsquelle unverändert bleibt.

46. Verfahren gemäß dem voranstehenden Patentanspruch 44, **dadurch gekennzeichnet, dass** die Frequenz und Phase der Pulsation des Röhrenstromes auf die Drehfrequenz der Drehanode derart abgestimmt wird, dass die Bewegung eines Quellengitters zur Bestimmung der Phasenverschiebung nachgebildet wird.

47. Verfahren gemäß dem voranstehenden Patentanspruch 40, **dadurch gekennzeichnet, dass** zur Erzeugung des Bündels von kohärenten Strahlen ein Elektronenstrahl (14) auf der Anodenoberfläche (12) entsprechend den Gitterlinien (13) eines röntgenoptischen Quellengitters bewegt wird, wobei die Gitterlinien des simulierten Quellengitters relativ zum Phasengitter (G₁) ortsfest bleiben.

48. Verfahren gemäß dem voranstehenden Patentanspruch 40, **dadurch gekennzeichnet, dass** zur Erzeugung des Bündels von kohärenten Strahlen (Sᵢ) ein Elektronenstrahl (14) auf der Anodenoberfläche (12) entsprechend den Gitterlinien eines röntgenoptischen Quellengitters bewegt wird, wobei eine Bewegung der Gitterlinien des simulierten Quellengitters relativ zum Phasengitter (G₁) zur Bestimmung der Phasenverschiebung erzeugt wird.

## Claims

1. Focus-detector arrangement of an X-ray apparatus (1) for generating projective or tomographic phase contrast images of an examination object (7, P), at least consisting of:
a radiation source (2) which is arranged on a first side of the examination object (7, P) and has a bundle of coherent rays (Si) having a grating-like origin,
a phase grating (G₁) which is arranged in the beam path on the opposite side of the examination object and which causes adjacent coherent rays to diffract and thereby generates an interference pattern of the X-ray radiation that is dependent on the phase shift of subregions of the examination object in a defined energy range of the X-ray radiation, and
an analyzer-detector system (G₂, D₁) which detects at least the interference pattern generated by the phase grating (G₁) in terms of its spatial intensity distribution (I) for determining a spatial phase shift (ϕ),
**characterised by** an anode (12) which generates the bundle of coherent rays (Sᵢ) having a grating-like origin and has regions (13) which are arranged in a strip shape and have different radiation emission and which run parallel to the grating lines of the phase grating (G₁) .

2. Focus-detector arrangement according to the preceding claim, **characterised in that** at least the surface of the anode (12) has regions of different material which are arranged in a strip shape in the area of an electron beam focal point generated for the purpose of operating the radiation source (2).

3. Focus-detector arrangement according to the preceding claim 2, **characterised in that** means (17.1, 17.2, 18.1, 18.2) are provided for shifting the anode (12), preferably vertically to the strip longitudinal direction.

4. Focus-detector arrangement according to the preceding claim 2, **characterised in that** means are provided for shifting the regions of different material arranged in a strip shape on the anode (12), preferably vertically to the strip longitudinal direction.

5. Focus-detector arrangement according to one of the preceding claims 1 to 4, **characterised in that** an electron mask (15) having strip-like apertures is arranged between cathode and anode (12), which apertures are mapped on the anode (12) and thereby lead to strip-like regions (13) of different radiation emission on the anode (12).

6. Focus-detector arrangement according to the preceding claim 5, **characterised in that** at least one optoelectronic lens (17.1, 17.2) is arranged between the electron mask (15) and the anode (12).

7. Focus-detector arrangement according to one of the preceding claims 6 to 7, **characterised in that** at least one optoelectronic lens is arranged between cathode and electron mask (15).

8. Focus-detector arrangement according to one of the preceding claims 6 to 7, **characterised in that** at least one optoelectronic lens is a magnetic field lens.

9. Focus-detector arrangement according to one of the preceding claims 6 to 7, **characterised in that** at least one optoelectronic lens is an electric field lens (17.1, 17.2).

10. Focus-detector arrangement according to one of the preceding claim 5 to 9, **characterised in** t h a t means are provided for shifting the electron mask, preferably vertically to the strip longitudinal direction.

11. Focus-detector arrangement according to one of the preceding claims 5 to 10, **characterised in that** means are provided for varying at least one optoelectronic lens which effects a shift in the mask mapping on the anode (12), preferably vertically to the strip longitudinal direction.

12. Focus-detector arrangement according to one of the preceding claims 1 to 11, **characterised in that** the anode (12) has peaks (20) and troughs (19) arranged in a strip shape at least in the region of an electron beam focal point generated for the purpose of operating the radiation source (2).

13. Focus-detector arrangement according to the preceding claim 12, **characterised in that** the waveform of the peaks (20) and troughs (19) is embodied in a wave shape, preferably in a sinusoidal shape.

14. Focus-detector arrangement according to the preceding claim 12, **characterised in that** the waveform of the peaks (20) and troughs (19) is embodied in a sawtooth shape.

15. Focus-detector arrangement according to the preceding claim 12, **characterised in that** the waveform of the peaks (20) and troughs (19) is embodied in a trapezoidal shape.

16. Focus-detector arrangement according to the preceding claim 12, **characterised in that** the waveform of the peaks (20) and troughs (19) is embodied in a rectangular shape.

17. Focus-detector arrangement according to one of the preceding claims 2 to 16, **characterised in that** the anode (12) is embodied as a rotating anode.

18. Focus-detector arrangement according to one of the preceding claims 2 to 16, **characterised in that** the rotating anode (12) has strips aligned in the direction of rotation.

19. Focus-detector arrangement according to the preceding claim 18, **characterised in that** the strips are arranged on a cone-shaped envelope surface of the rotating anode.

20. Focus-detector arrangement according to the preceding claim 18, **characterised in that** the strips are arranged on a cylinder-shaped envelope surface of the rotating anode.

21. Focus-detector arrangement according to one of the preceding claims 18 to 20, **characterised in that** :
the strips of the rotating anode have a directional component that is axial to the rotation axis of the rotating anode and
means are provided for generating and controlling a stroboscope-like pulsation of the tube current.

22. Focus-detector arrangement according to the preceding claim 21, **characterised in that** means are provided for tuning frequency and phase of the pulsation of the tube current and the rotational speed in such a way that the position of the strips of different material remains unchanged at the maximum of the tube current relative to the radiation source.

23. Focus-detector arrangement according to the preceding claim 21, **characterised in that** means are provided for tuning frequency and phase of the pulsation of the tube current and the rotational speed in such a way that the position of the strips of different material illuminated by the electron beam migrates at the maximum of the tube current relative to the radiation source, preferably incrementally, in the direction of rotation for measuring the phase shift in the case of a stationary phase grating and stationary analysis grating.

24. Focus-detector arrangement according to one of the preceding claims 21 to 23, **characterised in that** the strips (13) are arranged in parallel with the grating lines of the phase grating.

25. Focus-detector arrangement according to one of the preceding claims 17 or 19 to 24,
**characterised in that** the strips (13) are arranged at an angle, preferably at 45°, to the radial direction.

26. Focus-detector arrangement according to the preceding claim 1, **characterised in that** the radiation source (2) has means for generating and deflecting a bundled electron beam, wherein the electron beam is moved on an anode surface along at least one imaginary grating line.

27. Focus-detector arrangement according to the preceding claim 26, **characterised in that** a plurality of grating lines are provided and the electron beam jumps from grating line to grating line.

28. Focus-detector arrangement according to one of the preceding claims 26 to 27, **characterised in that** the grating lines are spaced apart from one another at intervals which are integral multiples of a basic spacing.

29. Focus-detector arrangement according to one of the preceding claims 26 to 28, **characterised in that** a rotating anode is provided.

30. Focus-detector arrangement according to the preceding claim 29, **characterised in that** the rotating anode has a cone-shaped anode surface and the lines on said surface are aligned radially with respect to the rotational axis of the rotating anode.

31. Focus-detector arrangement according to the preceding claim 29, **characterised in that** the rotating anode has a cone-shaped anode surface and the lines on said surface are aligned tangentially with respect to the rotational axis of the rotating anode.

32. Focus-detector arrangement according to the preceding claim 29, **characterised in that** the rotating anode has a cylinder-shaped anode surface and the lines on said surface are aligned in parallel with the rotational axis.

33. Focus-detector arrangement according to the preceding claim 29, **characterised in that** the rotating anode has a cylinder-shaped anode surface and the lines on said surface are aligned vertically with respect to the rotational axis.

34. Focus-detector arrangement according to one of the preceding claims 30 to 33, **characterised in that** the lines are aligned obliquely with respect to the rotational axis and to the radial direction.

35. Focus-detector arrangement according to one of the preceding claims 26 to 34, **characterised in that** the period of a revolution of the electron beam is small with factor 1/2 - 1/10, preferably very small with factor <1/10, relative to the scan period of the detector in the analysis-detector system.

36. Focus-detector arrangement according to one of the preceding claims 26 to 35, **characterised in that** the means (17.1, 17.2, 18.1, 18.2) for deflecting the electron beam are embodied in such a way that the movement of a source grating is simulated for determining the phase shift.

37. X-ray system for generating projective phase contrast images, **characterised in that** it has a focus-detector arrangement according to one of the preceding claims 1 to 36.

38. X-ray C-arm system for generating projective or tomographic phase contrast images,
**characterised in that** it has a focus-detector arrangement according to one of the preceding claims 1 to 36.

39. X-ray CT system for generating tomographic phase contrast images, **characterised in that** it has a focus-detector arrangement according to one of the preceding claims 1 to 36.

40. Method for generating projective or tomographic X-ray phase contrast images of an examination object with the aid of a focus-detector arrangement according to one of claims 1 to 32, containing the radiation source (2), the phase grating (G₁) and the analysis-detector system (G₂, D₁), wherein the bundle of coherent rays having a grating-like origin is generated by means of the anode (12) which has regions having different radiation emission which are arranged in a strip shape and run parallel to the grating lines of the phase grating (G₁).

41. Method according to the preceding claim 40,
**characterised in that** the strips (13) of different radiation emission are generated by means of regions of different material which are arranged in a strip shape.

42. Method according to one of the preceding claims 40 to 41, **characterised in that** the strips (13) of different radiation emission are generated by means of regions of different height (20) and depth (19) which are arranged in a strip shape.

43. Method according to one of the preceding claims 40 to 41, **characterised in that** a rotating anode is used.

44. Method according to the preceding claim 43,
**characterised in that** :
the strips of the rotating anode are operated by means of a directional component that is axial to the rotational axis of the rotating anode and
the tube current is pulsed in a stroboscope-like manner.

45. Method according to the preceding claim 44,
**characterised in that** the frequency and phase of the pulsation of the tube current is tuned to the rotational frequency of the rotating anode in such a way that the position of the strips of different radiation emission remains unchanged at the maximum of the tube current relative to the radiation source.

46. Method according to the preceding claim 44, **characterised in that** the frequency and phase of the pulsation of the tube current is tuned to the rotational frequency of the rotating anode in such a way that the movement of a source grating is simulated for the purpose of determining the phase shift.

47. Method according to the preceding claim 40, **characterised in that** an electron beam (14) is moved on the anode surface (12) according to the grating lines (13) of an X-ray optical source grating for the purpose of generating the bundle of coherent rays, wherein the grating lines of the simulated source grating remain stationary relative to the phase grating (G₁).

48. Method according to the preceding claim 40, **characterised in that** an electron beam (14) is moved on the anode surface (12) according to the grating lines of an X-ray optical source grating for the purpose of generating the bundle of coherent rays (Sᵢ), wherein a movement of the grating lines of the simulated source grating relative to the phase grating (G₁) is generated for the purpose of determining the phase shift.

## Revendications

1. Agencement foyer-détecteur d'un appareil ( 1 ) de rayons X pour la production de prises de vue projectives ou tomographiques à contraste de phase d'un objet ( 7, P ) à examiner, constitué au moins de :
une source ( 2 ) de rayonnement, qui est montée d'un premier côté de l'objet ( 7, P ) à examiner et qui produit un faisceau de rayons ( Sᵢ ) cohérents ayant une origine du type réseau,
un réseau ( G₁ ) de phase, qui est disposé dans le trajet des rayons du deuxième côté opposé de l'objet à examiner, qui porte à la diffraction des rayons cohérente voisins et produit ainsi, dans un certain domaine d'énergie du rayonnement X, un motif d'interférences dépendant du déphasage de sous-parties de l'objet ( 7, P ) à examiner, et
un système ( G₂, D₁ ) analyse-détecteur qui, pour la détermination d'un déphasage ( ϕ ) spatial, détecte au moins le motif d'interférences produit par le réseau ( G₁ ) de phase en ce qui concerne sa répartition ( I ) spatiale d'intensité,
**caractérisé par** une anode ( 12 ) qui produit le faisceau de rayons ( Sᵢ ) cohérent ayant une origine du type réseau et qui a des parties ( 13 ) disposées en forme de bande et ayant une émission de rayonnement différente, qui s'étendent parallèlement aux raies du réseau ( G₁ ) de phase.

2. Agencement foyer-détecteur suivant la revendication 1 précédente, **caractérisé en ce qu'**au moins la surface de l'anode ( 12 ) a des parties disposées en forme de bande en matériau différent dans la partie d'une tache focale d'un faisceau d'électrons produit lorsque la source ( 2 ) de rayonnement fonctionne.

3. Agencement foyer-détecteur suivant la revendication 2 précédente, **caractérisé en ce qu'**il est prévu des moyens ( 17.1, 17.2, 18.1, 18.2 ) de décalage de l'anode ( 12 ), de préférence perpendiculairement à la direction longitudinale des bandes.

4. Agencement foyer-détecteur suivant la revendication 2 précédente, **caractérisé en ce qu'**il est prévu des moyens de décalage de parties disposées en forme de bande en matériau différent sur l'anode, de préférence perpendiculairement à la direction longitudinale des bandes.

5. Agencement foyer-détecteur suivant l'une des revendications 1 à 4 précédentes, **caractérisé en ce qu'**il est disposé entre cathode et anode ( 12 ) un masque ( 15 ) d'électrons, ayant des ouvertures en forme de bandes, qui sont reproduites sur l'anode ( 12 ) et qui donnent ainsi sur l'anode ( 12 ) des parties ( 13 ) en forme de bandes ayant une émission de rayonnement différente.

6. Agencement foyer-détecteur suivant la revendication 5 précédente, **caractérisé en ce qu'**au moins une lentille ( 17.1, 17.2 ) en optoélectronique est disposée entre le masque ( 15 ) d'électron et l'anode ( 12 ).

7. Agencement foyer-détecteur suivant l'une des revendications 5 à 6 précédentes, **caractérisé en ce qu'**au moins une lentille en optoélectronique est disposée entre la cathode et le masque ( 15 ) d'électrons.

8. Agencement foyer-détecteur suivant l'une des revendications 6 à 7 précédentes, **caractérisé en ce qu'**au moins une lentille optoélectronique est une lentille magnétique de champ.

9. Agencement foyer-détecteur suivant l'une des revendications 6 à 7 précédentes, **caractérisé en ce qu'**au moins une lentille en optique électronique est une lentille ( 17.1, 17.2 ) électrique de champ.

10. Agencement foyer-détecteur suivant l'une des revendications 5 à 9 précédentes, **caractérisé en ce qu'**il est prévu des moyens de déplacement du masque d'électrons, de préférence perpendiculairement à la direction longitudinale des bandes.

11. Agencement foyer-détecteur suivant l'une des revendications 5 à 10 précédentes, **caractérisé en ce qu'**il est prévu des moyens de modification d'au moins une lentille optoélectronique, qui provoque un déplacement de la reproduction du masque sur l'anode ( 12 ), de préférence perpendiculairement à la direction longitudinale des bandes.

12. Agencement foyer-détecteur suivant l'une des revendications 1 à 11 précédentes, **caractérisé en ce que** l'anode ( 12 ) a, au moins dans une partie d'une tache focale, des hauts ( 20 ) et des creux ( 19 ) disposés en forme de bandes.

13. Agencement foyer-détecteur suivant la revendication 12 précédente, **caractérisé en ce que** le tracé des hauts ( 20 ) et des creux ( 19 ) est ondulé en étant de préférence sinusoïdal.

14. Agencement foyer-détecteur suivant la revendication 12 précédente, **caractérisé en ce que** le tracé des hauts ( 20 ) et des creux ( 19 ) est en dent de scie.

15. Agencement foyer-détecteur suivant la revendication 12 précédente, **caractérisé en ce que** le tracé des hauts ( 20 ) et des creux ( 19 ) est trapézoïdal.

16. Agencement foyer-détecteur suivant la revendication 12 précédente, **caractérisé en ce que** le tracé des hauts ( 20 ) et des creux ( 19 ) est rectangulaire.

17. Agencement foyer-détecteur suivant l'une des revendications 2 à 16 précédentes, **caractérisé en ce que** l'anode ( 12 ) est constituée sous la forme d'une anode tournante.

18. Agencement foyer-détecteur suivant l'une des revendications 2 à 16 précédentes, **caractérisé en ce que** l'anode tournante à des bandes dirigées dans le sens de rotation.

19. Agencement foyer-détecteur suivant la revendication 18 précédente, **caractérisé en ce que** les bandes sont disposées sur une surface latérale conique de l'anode tournante.

20. Agencement foyer-détecteur suivant la revendication 18 précédente, **caractérisé en ce que** les bandes sont disposées sur une surface latérale cylindrique de l'anode tournante.

21. Agencement foyer-détecteur suivant l'une des revendications 18 à 20 précédentes, **caractérisé en ce que** :
les bandes de l'anode tournante ont une composante de direction axiale par rapport à l'axe de rotation de l'anode tournante, et
il est prévu des moyens de production et de commande d'une pulsation stroboscopique de l'intensité du courant anodique.

22. Agencement foyer-détecteur suivant la revendication 21 précédente, **caractérisé en ce qu'**il est prévu des moyens d'accord de la fréquence et de la phase de la pulsation de l'intensité du courant anodique et de la vitesse de rotation de manière à ce que la position des bandes en matériau différent reste inchangée par rapport à la source de rayonnement au maximum de l'intensité du courant anodique.

23. Agencement foyer-détecteur suivant la revendication 21 précédente, **caractérisé en ce qu'**il est prévu des moyens d'accord de la fréquence et de la phase de la pulsation de l'intensité du courant anodique et de la vitesse de rotation de manière à ce que la position de bandes en matériau différent éclairées par le faisceau d'électrons se déplace, pour un réseau de phase fixe et un réseau d'analyse fixe au maximum de l'intensité du courant anodique, par rapport à la source de rayonnement, de préférence pas à pas dans le sens de rotation pour la mesure du déphasage.

24. Agencement foyer-détecteur suivant l'une des revendications 21 à 23 précédentes, **caractérisé en ce que** les bandes ( 13 ) sont disposées parallèlement aux raies du réseau de phase.

25. Agencement foyer-détecteur suivant l'une des revendications 17 ou 19 à 24 précédentes, **caractérisé en ce que** les bandes ( 13 ) sont disposées en faisant un angle de préférence de 45° par rapport à la direction radiale.

26. Agencement foyer-détecteur suivant la revendication 1 précédente, **caractérisé en ce que** la source ( 2 ) de rayonnement a des moyens de production et de déviation d'un faisceau d'électrons focalisé, le faisceau d'électrons se déplaçant sur une surface de l'anode le long d'au moins une raie imaginaire du réseau.

27. Agencement foyer-détecteur suivant la revendication 26 précédente, **caractérisé en ce qu'**il est prévu plusieurs raies du réseau et le faisceau d'électrons saute d'une raie du réseau à l'autre.

28. Agencement foyer-détecteur suivant l'une des revendications 26 à 27 précédentes, **caractérisé en ce que** les raies du réseau ont des intervalles entre elles qui représentent un multiple en nombre entier d'une distance de base.

29. Agencement foyer-détecteur suivant l'une des revendications 26 à 28 précédentes, **caractérisé en ce qu'**il est prévu une anode tournante.

30. Agencement foyer-détecteur suivant la revendication 29 précédente, **caractérisé en ce que** l'anode tournante à une surface d'anode conique et les lignes sur cette surface sont dirigées radialement par rapport à l'axe de rotation de l'anode tournante.

31. Agencement foyer-détecteur suivant la revendication 29 précédente, **caractérisé en ce que** l'anode tournante à une surface d'anode conique et les lignes sur cette surface sont dirigées tangentiellement à l'axe de rotation de l'anode tournante.

32. Agencement foyer-détecteur suivant la revendication 29 précédente, **caractérisé en ce que** l'anode tournante à une surface d'anode cylindrique et les lignes sur cette surface sont dirigées parallèlement à l'axe de rotation.

33. Agencement foyer-détecteur suivant la revendication 29 précédente, **caractérisé en ce que** l'anode de rotation à une surface d'anode cylindrique et les lignes sur cette surface sont dirigées perpendiculairement à l'axe de rotation.

34. Agencement foyer-détecteur suivant l'une des revendications 30 à 33 précédentes, **caractérisé en ce que** les lignes sont inclinées par rapport à l'axe de rotation et par rapport à la direction radiale.

35. Agencement au foyer-détecteur suivant l'une des revendications 26 à 34 précédentes, **caractérisé en ce que** la période d'un tour du faisceau d'électrons est plus petite, d'un facteur 1/2 à 1/10, de préférence bien plus petite, d'un facteur inférieur à 1/10, que la période de balayage du détecteur du système analyse-détecteur.

36. Agencement foyer-détecteur suivant l'une des revendications 26 à 35 précédentes, **caractérisé en ce que** les moyens ( 17.1, 17.2, 18.1, 18.2 ) de déviation du faisceau d'électrons sont conformés de manière à ce que le mouvement d'un réseau de source soit reproduit pour la détermination du déphasage.

37. Système de rayon X pour la production de prises de vue projectives à contraste de phase, **caractérisé en ce qu'**il comporte un agencement foyer-détecteur suivant l'une des revendications 1 à 36 précédentes.

38. Système de rayon X en arceau en C de production de prises de vue projectives ou tomographiques à contraste de phase, **caractérisé en ce qu'**il comporte un agencement foyer-détecteur suivant l'une des revendications 1 à 36 précédentes.

39. Système de rayon X CT de production de prises de vue tomographique à contraste de phase, **caractérisé en ce qu'**il comporte un agencement foyer-détecteur suivant l'une des revendications 1 à 36 précédentes.

40. Procédé de production de prises de vue projectives ou tomographiques de rayons X à contraste de phase d'un objet à examiner à l'aide d'un agencement foyer-détecteur suivant l'une des revendications 1 à 32, comportant la source ( 2 ) de rayonnement, le réseau ( G₁ ) de phase, le système ( G₂, D₁ ) d'analyse-détecteur et l'anode ( 12 ), dans lequel le faisceau de rayons cohérents à origine de type réseau est produit par l'anode ( 12 ) qui a des parties en forme de bande ayant une émission de rayonnement différente, qui s'étendent parallèlement aux raies du réseau ( G₁ ) de phase.

41. Procédé suivant la revendication 40 précédente, **caractérisé en ce que** l'on produit des bandes ( 13 ) ayant une émission de rayonnement différente par des parties disposées en forme de bande en matériau différent.

42. Procédé suivant l'une des revendications 40 à 41 précédentes, **caractérisé en ce qu'**on produit des bandes ( 13 ) ayant une émission de rayonnement différente par des parties disposées en forme de bande de hauteur ( 20 ) et de profondeur ( 19 ) différente.

43. Procédé suivant l'une des revendications 40 à 41 précédentes, **caractérisé en ce qu'**on utilise une anode tournante.

44. Procédé suivant la revendication 43 précédente, **caractérisé en ce que** :
les bandes de l'anode tournante fonctionnent en ayant une composante de direction axiale par rapport à l'axe de rotation de l'anode tournante et
on pulse stroboscopiquement l'intensité du courant anodique.

45. Procédé suivant la revendication 44 précédente, **caractérisé en ce que** l'on accorde la fréquence et la phase de la pulsation de l'intensité du courant anodique à la fréquence de rotation de l'anode tournante de manière à ce que la position des bandes ayant une émission de rayonnement différente reste inchangée par rapport à la source de rayonnement au maximum de l'intensité du courant anodique.

46. Procédé suivant la revendication 44 précédente, **caractérisé en ce que** l'on accorde la fréquence et la phase de la pulsation de l'intensité du courant anodique à la fréquence de rotation de l'anode tournante de manière à reproduire le déplacement d'un réseau source pour la détermination du déphasage.

47. Procédé suivant la revendication 40 précédente, **caractérisé en ce que**, pour la production du faisceau de rayons cohérents, on déplace un faisceau ( 14 ) d'électrons sur la surface ( 12 ) de l'anode conformément aux raies ( 13 ) d'un réseau source en optique de rayons X, les raies du réseau source simulé restant fixes par rapport au réseau ( G₁ ) de phase.

48. Procédé suivant la revendication 40 précédente, **caractérisé en ce que**, pour la production du faisceau de rayons ( Si ) cohérents, on déplace un faisceau ( 14 ) d'électrons sur la surface ( 12 ) de l'anode conformément aux raies d'un réseau source en optique de rayons X, un déplacement des raies du réseau source simulé par rapport au réseau ( G₁ ) de phase étant produit pour la détermination du déphasage.
